# EUROPEAN PATENT SPECIFICATION

(11) **EP 3 726 533 B1**
(45) Date of publication and mention of the grant of the patent: **11.11.2026**
(21) Application number: 20169637.4
(22) Date of filing: 15.04.2020
(51) Int. Cl.: G16H 15/00, G16H 10/60, G16H 40/63

(54) **SEPSIS AUTOMATED REPORTING SYSTEM**
AUTOMATISIERTES SEPSISMELDESYSTEM
SYSTÈME DE SIGNALEMENT AUTOMATISÉ DE SEPTICÉMIE

(30) Priority: 19.04.2019 US 201962836321 P
(43) Date of publication of application: 21.10.2020
(73) Proprietor: Hill-Rom Services, Inc., Batesville, IN 47006-9167 (US)
(72) Inventor: CHUNG, Chiew Yuan, Batesville, IN 47006-9167 (US); FITZGIBBONS, Stacey A, Batesville, IN 47006-9167 (US); KAYSER, Susan, Batesville, IN 47006-9167 (US); RIORDAN, Matthew McCormick, Batesville, IN 47006-9167 (US); SHI, Yuan, Batesville, IN 47006-9167 (US); URRUTIA, Eugene, Batesville, IN 47006-9167 (US); ZAPFE, Lori Ann, Batesville, IN 47006-9167 (US)
(74) Representative: Reddie & Grose LLP

(56) References cited:
- EP-B1- 1 623 666
- WO-A1-2017/189957
- WO-A1-2019/028448
- CN-A- 104 536 703
- US-A1- 2011 137 852
- US-A1- 2015 025 329
- US-A1- 2016 128 646
- US-B2- 8 527 449

## Description

### INTRODUCTION

Sepsis is a life-threatening condition that occurs when the body's response to infection causes injury to its own tissues and organs. Sepsis develops when a pathogen is released into the bloodstream and causes inflammation throughout the entire body.

In early stages, it is difficult to differentiate sepsis from other diseases because certain symptoms of sepsis, such as fever, increased heart rate, and breathing rate, mimic the symptoms of other diseases. The ability to detect sepsis at its earliest stages is critical because early sepsis is usually reversible with antibiotics, fluids, and other supportive medical interventions. However, as time progresses the risk of dying increases substantially.

Hospitals and other healthcare facilities must satisfy Medicare and Medicaid specified requirements when treating sepsis patients. The extraction of information regarding compliance with these requirements is time-consuming, prone to human error, and not efficient. Additionally, information on the diagnosis, treatment, condition, and outcome of sepsis patients should be extracted promptly so that caregivers can better understand sepsis risk factors, monitor sepsis protocol compliance status, and minimize hospital costs for sepsis treatment. US 2015/025329 A1 relates to a patient care surveillance system comprising a data store operable to receive and store clinical and non-clinical data associated with at least one patient, a user interface configured to receive user input of current information related to at least one patient, a monitor configured to sense at least one parameter associated with at least one patient, and further configured to generate real-time patient monitor data, a data analysis module configured to access the data store and analyze the clinical and non-clinical data, receive and analyze the current information and real-time patient monitor data, and identify at least one adverse event associated with the care of at least one patient, and a data presentation module operable to present information associated with at least one adverse event to a healthcare professional, the information including contextual information associated with the adverse event. US 2011/137852 A1 describes a computer-implemented sepsis alerting method. The method involves automatically extracting with a computer system, from records maintained for a patient under care in a healthcare facility, information from an electronic medical record, and obtaining with the computer system information about real-time status of the patient. The method also involves using the information from the electronic medical record and the information about the real-time status to determine whether the patient is likely to be suffering from dangerous probability of sepsis, using information from the electronic medical record to determine whether treatment for sepsis is already being provided to the patient, and electronically alerting a caregiver over a network if it is determined that a potentially dangerous level of sepsis exists and that treatment for sepsis is not already being provided.

The present invention is defined by the appended independent claim.

In one embodiment, the sepsis automated reporting system is cloud based and is accessible by a web portal that provides a single sign-on configuration application. In another embodiment, the sepsis automated reporting system is accessible by an intranet portal of a local area network that provides a single sign-on configuration application.

The sepsis report is a patient investigation report that includes data from a single, septic patient in a healthcare facility. The patient investigation report enables selection of a vital sign parameter and clinical events in the parameter configuration box to generate a graph in the display area displaying the vital sign parameter and the clinical events over a period of time.

In another embodiment, the sepsis report is an institutional compliance report that includes data from a plurality of patients in a healthcare facility to assess and track compliance with one or more goals related to septic patient treatment in the healthcare facility. In one example, the one or more goals include time-based metrics.

In another embodiment, the sepsis report is an institutional insights report that includes data from a plurality of patients in a healthcare facility over a period of time to identify correlations between septic patient outcomes and clinical parameters. In one example, the institutional insights report enables selection of a department within the healthcare facility and at least one additional parameter in the parameter configuration box to generate a graph in the display area, the graph displaying the number of patients who acquired sepsis while admitted in the selected department and the selected additional parameter over a period of time.

The hospital information systems can include an admission, discharge, and transfer system, a lab system, a medication system, a nurse call system, a real-time locating system, and a mobile device platform. The patient monitoring and support devices can include at least one of a vital signs monitor, a bed, and a mattress pad device.

The sepsis report may be accessible via a plurality of workstations within a healthcare facility. In one embodiment, the communication module acquires the data using a Health Level Seven International messaging protocol.

In one embodiment, the sepsis report further includes a time configuration box where a date and time range are selectable. In one example, selections made in the parameter configuration box and the time configuration box enable customization of the sepsis reports.

In another embodiment, which does not form part of the present claimed invention, a non-transitory computer-readable storage medium stores instructions which when read and executed by one or more processors, cause the one or more processors to: generate a sepsis report that is accessible on one or more workstations; display a customizable display area in the sepsis report based on a selection of one or more parameters in a parameter configuration box, the customizable display area and the parameter configuration box both displayed in the sepsis report; and update the customizable display area in the sepsis report based on a selection of a different combination of parameters in the parameter configuration box.

In another embodiment, which does not form part of the present claimed invention, a device for displaying a sepsis report comprises: at least one processing unit; and a system memory storing instructions that, when executed by the at least one processing unit, cause the at least one processing unit to: display a sepsis report having at least one parameter configuration box, and a customizable display area, the at least one parameter configuration box including a plurality of selectable parameters related to sepsis diagnosis, treatment, and septic patient outcomes; and update the customizable display area in the sepsis report based on a selection of one or more selectable parameters in the parameter configuration box, the updated customizable display area and the parameter configuration box both being displayed in the sepsis report. The sepsis report can be a patient investigation report that includes data from a single, septic patient in a healthcare facility. In another example, the sepsis report is an institutional compliance report that includes data from a plurality of patients in a healthcare facility to assess and track compliance with one or more goals related to septic patient treatment in the healthcare facility. In a further example, the sepsis report is an institutional insights report that includes data from a plurality of patients in a healthcare facility over a period of time to identify correlations between septic patient outcomes and clinical parameters.

A variety of additional inventive aspects will be set forth in the description that follows. The inventive aspects can relate to individual features and to combinations of features. It is to be understood that both the forgoing general description and the following detailed description are exemplary and explanatory only and are not restrictive of the broad inventive concepts upon which the examples disclosed herein are based.

The invention will now be further described by way of example with reference to the accompanying drawings, in which:
FIG. 1 is a block diagram schematically illustrating a healthcare facility that includes a sepsis automated reporting system.
FIG. 2 is a block diagram schematically illustrating the inputs and outputs, and communications of the sepsis automated reporting system.
FIG. 3 is a schematic block diagram of the sepsis automated reporting system.
FIG. 4 is a schematic block diagram of a database storage.
FIG. 5 is a schematic block diagram of a report generator.
FIG. 6 illustrates a sepsis patient investigation report.
FIG. 7 illustrates a sepsis patient investigation report.
FIG. 8 illustrates a sepsis institutional insights report.
FIG. 9 illustrates another sepsis institutional insights report.
FIG. 10 illustrates another sepsis institutional insights report.
FIG. 11 illustrates another sepsis institutional insights report.
FIG. 12 illustrates another sepsis institutional insights report.
FIG. 13 illustrates another sepsis institutional insights report.
FIG. 14 illustrates another sepsis institutional insights report.
FIG. 15 illustrates another sepsis institutional insights report.
FIG. 16 illustrates another sepsis institutional insights report.
FIG. 17 illustrates another sepsis institutional insights report.
FIG. 18 illustrates a sepsis institutional compliance report.
FIG. 19 illustrates another sepsis institutional compliance report.
FIG. 20 illustrates another sepsis institutional insights report.
FIG. 21 illustrates another sepsis institutional insights report.
FIG. 22 illustrates another sepsis institutional insights report.
FIG. 23 illustrates another sepsis institutional insights report.
FIG. 24 illustrates another sepsis institutional insights report.
FIG. 25 illustrates another sepsis institutional insights report.
FIG. 26 illustrates a sepsis institutional compliance report.
FIG. 27 illustrates another sepsis institutional compliance report.
FIG. 28 illustrates another sepsis institutional compliance report.
FIG. 29 illustrates a sepsis patient investigation report.
FIG. 30 illustrates another sepsis patient investigation report.
FIG. 31 illustrates another sepsis patient investigation report.
FIG. 32 illustrates another sepsis patient investigation report.
FIG. 33 illustrates a sepsis institutional insights report.
FIG. 34 illustrates a sepsis patient investigation report.
FIG. 35 illustrates another sepsis patient investigation report.
FIG. 36 schematically illustrates the physical components of a computing device.

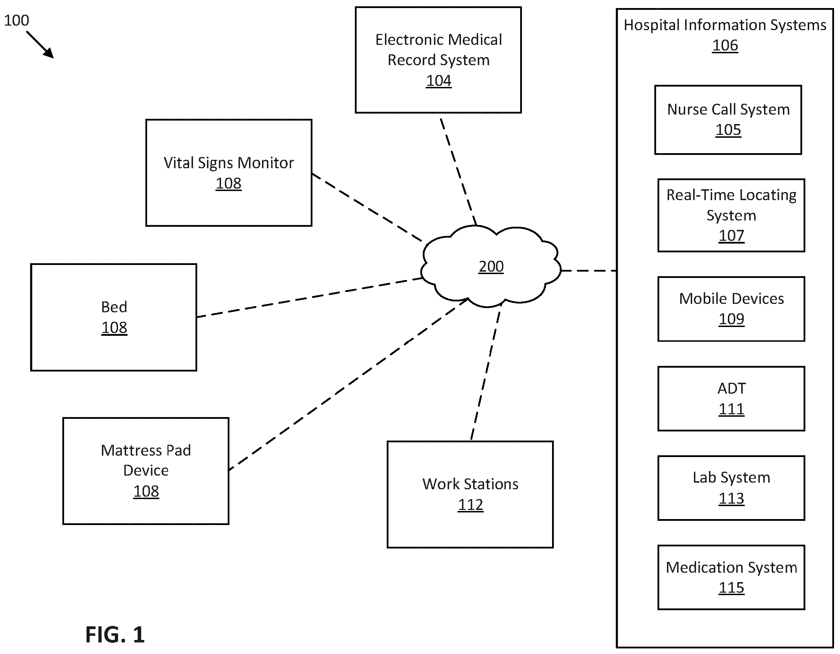

FIG. 1 is a block diagram schematically illustrating a healthcare facility 100 that includes a sepsis automated reporting system 200. As will be described in more detail, the sepsis automated reporting system 200 is a clinical decision support tool that automatically generates customizable sepsis reports that are accessible on one or more workstations 112.

The sepsis automated reporting system 200 is connected to an electronic medical record system 104, hospital information systems 106 including a nurse call system 105, a real-time locating system (RTLS) 107, and a mobile device platform 109, patient monitoring and support devices 108, and one or more workstation 112. In the embodiment illustrated in FIG. 1, the nurse call system 105, RTLS 107, and mobile device platform 109 are part of the hospital information systems 106. In alternative embodiments, the nurse call system 105, RTLS 107, and mobile device platform 109 are separate systems from the hospital information systems 106.

The sepsis reports are generated using data acquired by the sepsis automated reporting system 200 from the electronic medical record system 104, the hospital information systems 106, and, patient monitoring and support devices 108. The sepsis reports allow caregivers to track hospital sepsis protocol compliance, and provide data visualization and actionable insights to prioritize sepsis interventions. The sepsis reports can be used to determine the pain points of the healthcare facility 100, and prioritize areas for improvement. The sepsis reports can also be used to investigate an incident and track compliance with the hospital's sepsis prevention protocol. While the description herein refers to sepsis reports, it is contemplated that the sepsis automated reporting system 200 can be adapted to automatically generate additional types of medical reports including reports that monitor patient falls and/or patient deterioration within the healthcare facility 100.

In one embodiment, the sepsis automated reporting system 200 is a cloud based system that is hosted over the Internet. In this embodiment, the sepsis automated reporting system 200 is accessible from the workstations 112 via a web portal that provides a single sign-on configuration application.

In an alternative embodiment, the sepsis automated reporting system 200 is part of a local area network and is stored onsite in the healthcare facility 100. In this embodiment, the sepsis automated reporting system 200 is accessible from the workstations 112 via an intranet portal that provides a single sign-on configuration application.

The one or more workstations 112 can include stationary desktop computers, and portable computing devices such as smartphones, tablet computers, and the like. Although only one workstation 112 is depicted in FIG. 1, it is contemplated that the healthcare facility 100 can include a plurality of workstations 112.

The sepsis reports are accessible on the one or more workstations 112. The sepsis reports include a display area that is customizable based on a selection of one or more parameters in a parameter configuration box. The customizable display area and the parameter configuration box are both displayed in the sepsis report, and the display area is automatically updated based on the selection of the one or more parameters in the parameter configuration box. As will be described in more detail, the parameter configuration box includes a plurality of selectable parameters related to sepsis diagnosis, treatment, and septic patient outcomes.

The sepsis automated reporting system 200 also provides back office settings where an administrator can configure the rules for determining high/medium/low patient risk, and the types of notifications that are sent based on the determined risk. The back office settings can also allow the administrator to configure the rules for granting access to the sepsis reports.

The electronic medical record system 104 stores a plurality of electronic medical records (EMRs). Each EMR contains the medical and treatment history of a patient admitted to the healthcare facility 100. In some examples, the electronic medical record system 104 includes one or more systems developed and managed from Epic Systems Corporation, Cerner Corporation, Allscripts, and Medical Information Technology, Inc. (Meditech).

The hospital information systems 106 can include, in addition to the nurse call system 105, RTLS 107, and mobile device platform 109, an Admission, Discharge, and Transfer (ADT) system 111, a lab system 113, a medication system 115, and other hospital related systems. An ADT system provides real-time information on each patient admitted to the healthcare facility 100 including the patient's name, address, gender, room assignment within the healthcare facility 100, the date and time when admitted to and discharged from the healthcare facility 100, and whether the patient has been transferred to another room or department within the healthcare facility 100. The lab system monitors patient samples and lab results. The medication system monitors the medications prescribed to each patient within the healthcare facility 100.

The nurse call system 105 can be used by the sepsis automated reporting system 200 to collect data such as code calls or bed-based alerts sent through the nurse call system 105 in order to provide a complete timeline of relevant patient events that can be used by the sepsis automated reporting system 200 to generate the customizable sepsis reports.

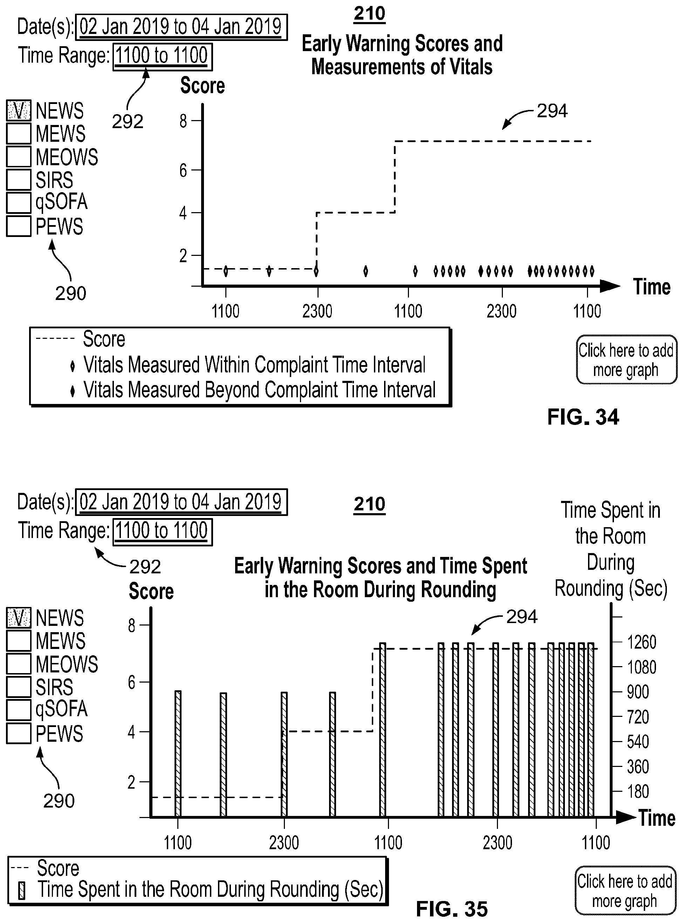

The RTLS 107 can be used by the sepsis automated reporting system 200 to collect data on caregiver time spent in the patient's room during rounding. For example, this data can be used to generate a patient investigation report 210 such as the one illustrated in FIG. 35.

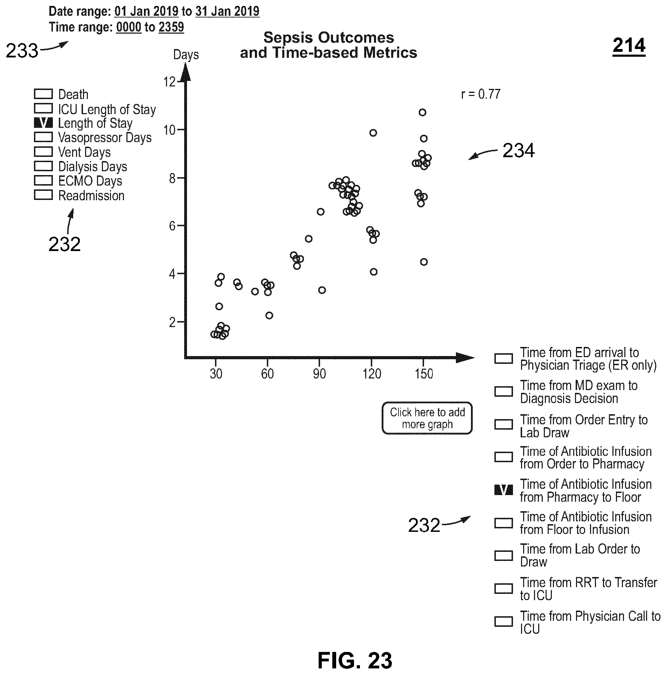

The mobile device platform 109 can be installed smartphone cellular devices used by caregivers in the healthcare facility 100. The mobile device platform 109 can be used by the sepsis automated reporting system 200 to collect data to obtain metrics relating to calls to the rapid response team (RRT) or physicians. For example, this data can be used to generate an institutional insights report 214 such as the one illustrated in FIG. 23.

The patient monitoring and support devices 108 include a continuous vital signs monitor, bed, and mattress pad devices. Each of the patient monitoring and support devices 108 is configured to detect and measure one or more vital signs of a patient admitted to the healthcare facility 100. For example, the vital signs monitor can be used to take vital signs such as temperature, heart rate, respiratory rate, blood pressure, pulse oximetry, and the like. In some examples, the vital signs monitor is a Connex^{®} Spot Monitor (CSM) or Connex^{®} Vital Signs Monitor (CVSM) available from Welch Allyn Inc., Skaneateles Falls, NY.

The bed measures the patient's weight and can also take the heart rate and respiratory rate of the patient. The bed also monitors the patient's motion and transmits an alarm to alert the caregiver when it is detected that the patient is exiting the bed without authorization. In some examples, the bed is a Centrella^{®} Smart+ bed, Progressa^{®} bed system, or VersaCare^{®} Med Surg Bed, each available from Hill-Rom Services, Inc., Batesville, IN.

The mattress pad device is configured to be placed under the mattress of a bed, such as the bed described above, and continuously monitors heart rate, respiratory rate, and motion to help identify early detection of patient deterioration, prevent falls, and prevent pressure ulcers. In some examples, the mattress pad device is an EarlySense^{®} system.

The sepsis automated reporting system 200 communicates with the electronic medical record system 104, the hospital information systems 106 including the nurse call system 105, RTLS 107, and mobile device platform 109, the patient monitoring and support devices 108, and workstations 112 through a wireless connection, a wired connection, or a combination of wireless and wired connections. Examples of wireless connections can include, without limitation, Wi-Fi communication devices that utilize wireless routers or wireless access points, cellular communication devices that utilize one or more cellular base stations, Bluetooth, ANT, ZigBee, medical body area networks, personal communications service (PCS), wireless medical telemetry service (WMTS), and other similar wireless communication devices and services.

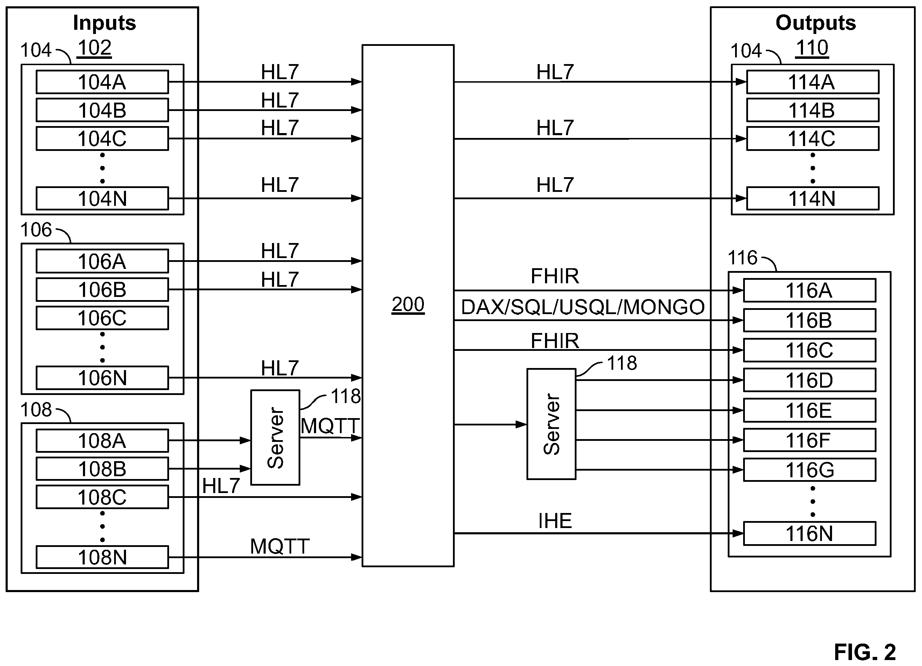

FIG. 2 is a block diagram schematically illustrating the inputs 102 and outputs 110 of the sepsis automated reporting system 200. The sepsis automated reporting system 200 retrieves inputs 104a-104n from the electronic medical record system 104, inputs 106a-106n from the hospital information systems 106, and inputs 108a-108n from the patient monitoring and support devices 108. In the embodiment illustrated in FIG. 2, the nurse call system 105, RTLS 107, and mobile device platform 109 are part of the hospital information systems 106.

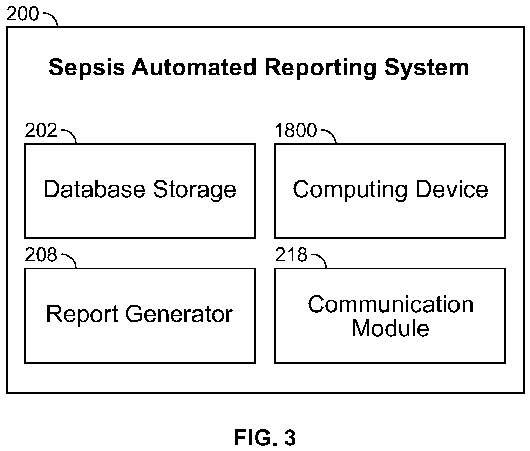

The inputs 104a-104n are directly retrieved by a communication module 218 (see FIG. 3) using a Health Level Seven International (HL7) messaging protocol that allows the data to be shared and processed in a uniform and consistent manner. Similarly, the inputs 106a-106n are directly retrieved by the communication module 218 over the HL7 data protocol.

The inputs 108a-108n can be directly retrieved by the communication module 218 using HL7 data standards. For example, inputs from the vital signs monitor can be retrieved using the HL7 standard. The inputs 108a-108n can also be retrieved by the communication module 218 using a Message Queuing Telemetry Transport (MQTT) messaging protocol. For example, inputs from the mattress pad devices such as the EarlySense^{®} system can be communicated over the MQTT standard. In some further embodiments, the inputs 108a-108n are indirectly retrieved by the communication module 218 using a secondary server 118. For example, the communication module 218 can communicate with a secondary server 118 such as the SmartSync^{™} system from Hill-Rom Services, Inc. to retrieve data from the beds such as the Centrella^{®} Smart+ bed, Progressa^{®} bed system, or VersaCare^{®} Med Surg Bed.

The sepsis automated reporting system 200 generates outputs 114a-114n for the electronic medical record system 104 and outputs 116a-116n for clinical user interfaces 116. At least one of the outputs 116a-116n is a sepsis report on a web portal or intranet portal that is accessible via the one or more workstations 112.

Outputs 114a-114n are directly sent to the electronic medical record system 104 using the HL7 messaging protocol. Outputs 116a-116n are directly sent to a clinical user interface 116 using Fast Healthcare Interoperability Resources (FHIR), Integrating the Healthcare Enterprise (IHE), or DAX/SQL/USQL/MONGO data formats. In some examples, the outputs 116a-116n are indirectly sent to a clinical user interface 116 using a secondary server 118.

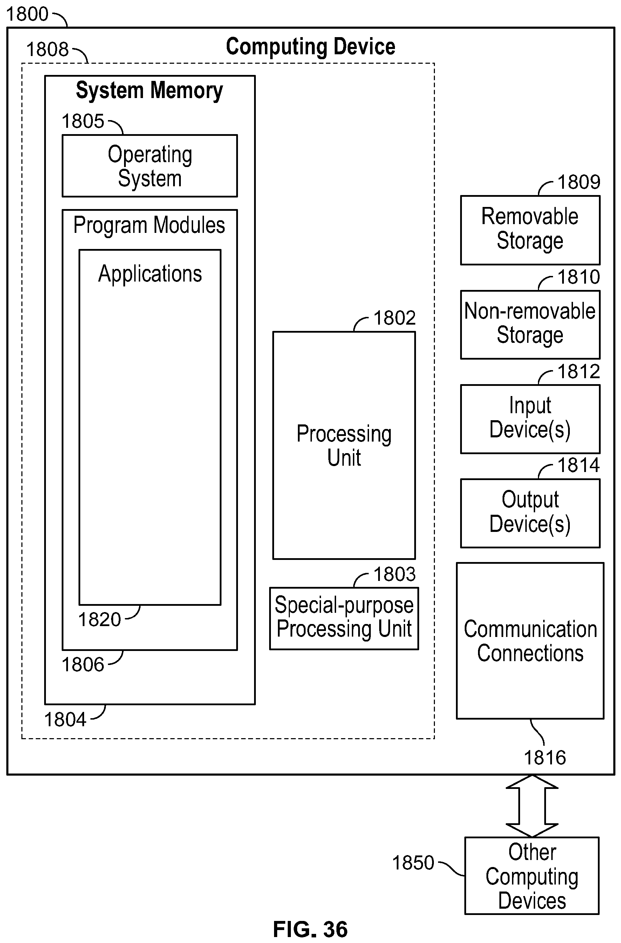

FIG. 3 is a schematic block diagram of the sepsis automated reporting system 200. The sepsis automated reporting system 200 includes a database storage 202, a report generator 208, the communication module 218, and a computing device 1800. The database storage 202 stores the data retrieved from the electronic medical record system 104, the hospital information systems 106 including the nurse call system 105, RTLS 107, and mobile device platform 109, and the patient monitoring and support devices 108. The report generator 208 uses the data stored in the database storage 202 to generate the sepsis reports. The communication module 218 enables the sepsis automated reporting system 200 to communicate with the electronic medical record system 104, the hospital information systems 106 including the nurse call system 105, RTLS 107, and mobile device platform 109, the patient monitoring and support devices 108, and workstations 112. The computing device is described in more detail with reference to FIG. 36.

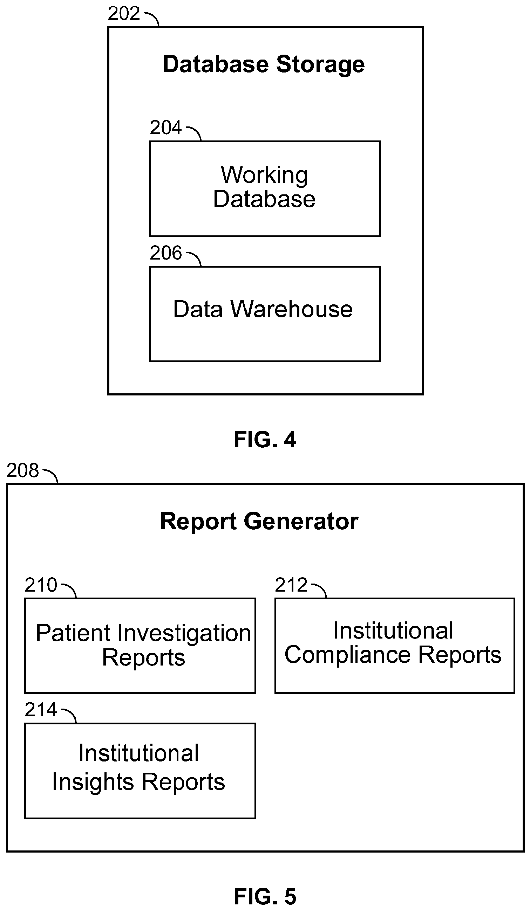

FIG. 4 is a schematic block diagram of the database storage 202 of the sepsis automated reporting system 200. As shown in FIG. 4, the database storage 202 includes a working database 204 and a separate data warehouse 206.

The working database 204 temporarily stores the data from the electronic medical record system 104, hospital information systems 106, and patient monitoring and support devices 108. The data in the working database 204 is used to trigger one or more rules and/or notifications. For example, patient early warning scores (EWS) when above a predetermined threshold trigger notifications to the caregiver and clinical tasks for the caregiver to perform. In certain examples, the working database 204 is a clinical data repository (CDR).

The data in the working database 204 is removed or erased after a predetermined event or period of time. For example, the data in the working database 204 is removed upon the patient's discharge from the healthcare facility 100 or upon a predetermined amount of time after the patient's discharge from the healthcare facility 100.

The data warehouse 206 stores the data long term from the electronic medical record system 104, the hospital information systems 106 including the nurse call system 105, RTLS 107, and mobile device platform 109, and the patient monitoring and support devices 108. The data stored in the data warehouse 206 is used by the report generator 208 to populate the various sepsis reports disclosed herein.

In one example embodiment, the data collected from the electronic medical record system 104, the hospital information systems 106 including the nurse call system 105, RTLS 107, and mobile device platform 109, and the patient monitoring and support devices 108 is stored simultaneously in both the working database 204 and the data warehouse 206. In an alternative example embodiment, the data collected from the electronic medical record system 104, the hospital information systems 106 including the nurse call system 105, RTLS 107, and mobile device platform 109, and patient monitoring and support devices 108 is first stored in the working database 204, and thereafter, is stored in the data warehouse 206.

FIG. 5 is a schematic block diagram of the report generator 208 of the sepsis automated reporting system 200. The report generator 208 automatically generates various sepsis reports by collecting data from the electronic medical record systems 104, the hospital information systems 106 including the nurse call system 105, RTLS 107, and mobile device platform 109, and patient monitoring and support devices 108. The sepsis reports generated by the report generator 208 are accessible via a web portal or intranet portal on the workstation 112. As shown in FIG. 5, the report generator 208 can automatically generate patient investigation reports 210, institutional compliance reports 212, and institutional insights reports 214.

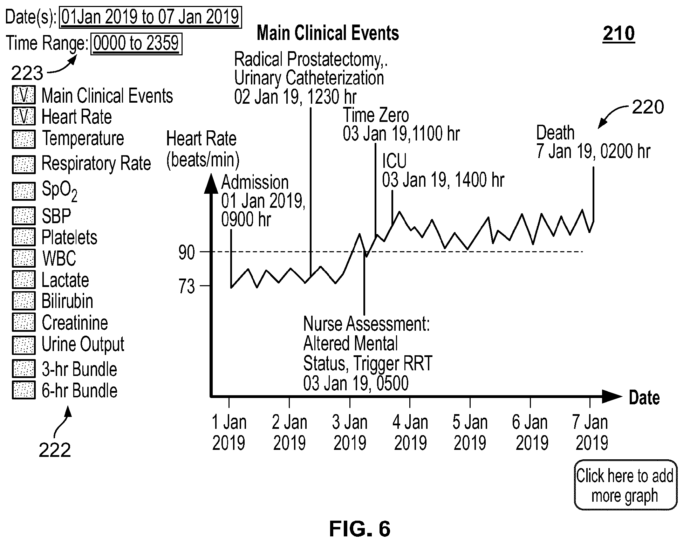

The patient investigation reports 210 include data from a single, septic patient for investigating data and events associated with the septic patient's stay in the healthcare facility 100. These reports can include data indicating compliance with a sepsis protocol for that patient. Additionally, the patient investigation reports 210 can allow caregivers to investigate events prior to the patient first developing sepsis, and thus may provide insight as to the possible causes for the patient developing sepsis. The patient investigation reports 210 also allow caregivers to investigate explanations for patient outcomes by providing data that monitors and tracks the patient's condition and treatment following sepsis diagnosis. Examples of patient investigation reports 210 are included in FIGS 6, 7, 29-32, 34, and 35.

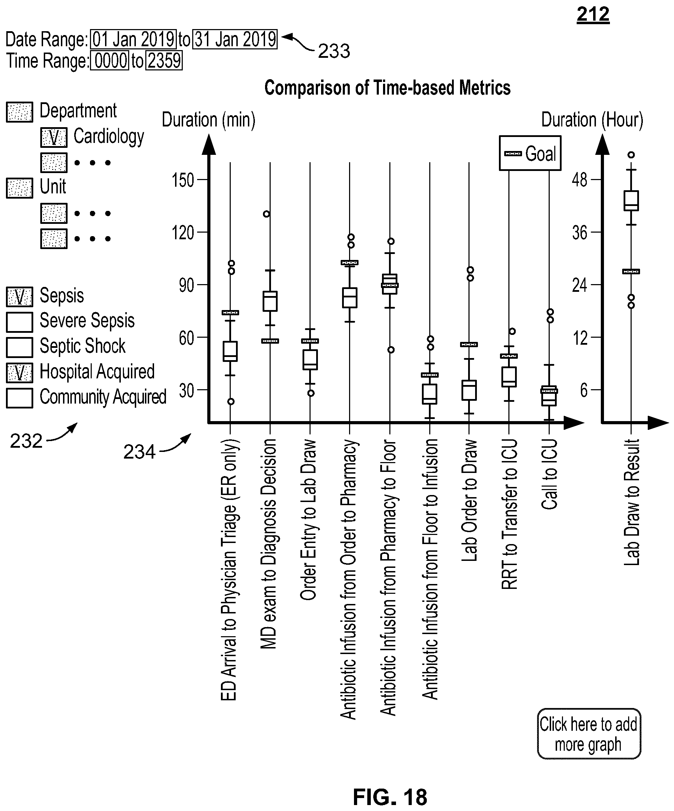

The institutional compliance reports 212 include compliance data across a number of patients to help an institution such as the healthcare facility 100 to assess and track compliance across different metrics, and can include configurations for one or more compliance goals. Thus, the institutional compliance reports 212 are institution level or unit wide reports that aggregate the data from individual patients and display information on whether bundle compliance and other forms of compliance were adhered to during sepsis evaluation and treatments. These reports allow caregivers to determine whether Medicare and Medicaid specified requirements were satisfied at the institution level for sepsis patient treatment. Examples of institutional compliance reports 212 are shown in FIGS. 18, 19, and 26-28.

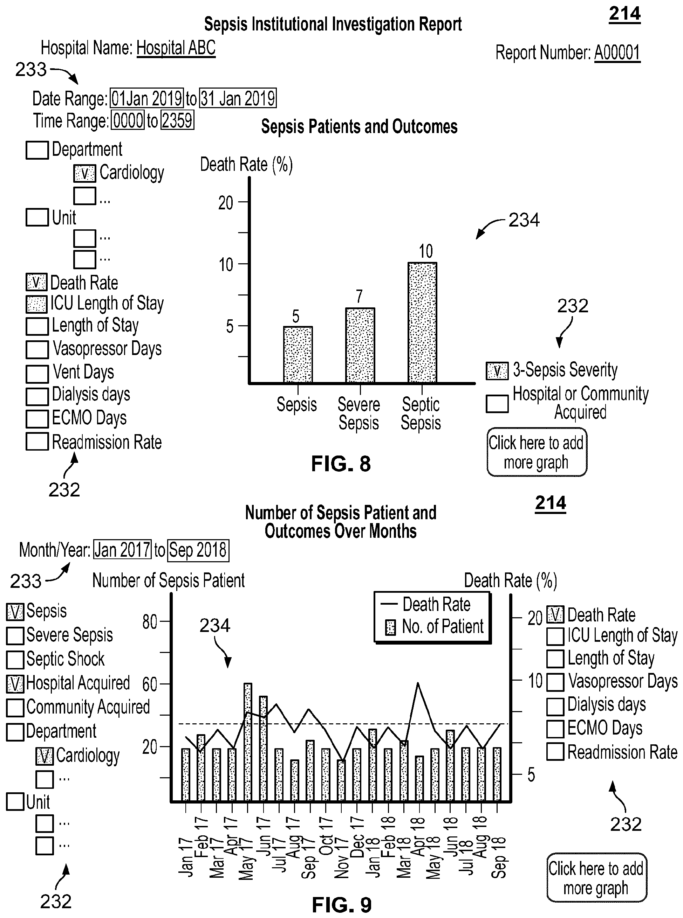

The institutional insights reports 214 include data across a number of patients over a defined period of time. These reports can be used to identify correlations between different clinical parameters and septic patient outcomes. The institutional insights reports 214 can provide insights allowing caregivers to determine how well early warning scores (EWSs) are predicting sepsis and how septic patient outcomes correlate with various clinical parameters. Examples of institutional insights reports 214 are included in FIGS. 8-17, 20-25, and 33.

FIGS. 6, 7, 29-32, 34, and 35 illustrate example patient investigation reports 210. As shown in these figures, the patient investigation reports 210 include at least one parameter configuration box 222 where one or more parameters are selectable for customizing a display area 220. The at least one parameter configuration box 222 includes a plurality of selectable parameters related to sepsis diagnosis, treatment, and septic patient outcomes. For example, the parameter configuration box 222 can include, without limitation, parameters such as main clinical events (and sub-parameters admission, surgery/procedures, nurse assessment/trigger of rapid response team (RRT) call, transfer to ICU, death, etc.), early warning score (EWS) (and sub-parameters NEWS, MEWS, MEOWS, SIRS, qSOFA, PEWS, etc.), heart rate, temperature, respiratory rate, SpO₂, systolic blood pressure (SBP), platelets, WBC, lactate, bilirubin, creatinine, and urine output. The parameter configuration box 222 also includes sepsis treatment events that are selectable for display such as 3-hr and 6-hr sepsis treatment bundles.

In the illustrative example of FIG. 6, main clinical events and a vital sign parameter (e.g., heart rate) are selected in the parameter configuration box 222, and the display area 220 includes a graph to display the vital sign parameter (e.g., heart rate) and main clinical events data over a period of time. While heart rate is selected as the vital sign parameter in the illustrative example of FIG. 6, additional vital signs parameters can be selected in the parameter configuration box 222 for display in the graph generated in the display area 220.

Additionally, the patient investigation reports 210 include a time configuration box 223 where a time range is selectable. Selections made in the parameter configuration box 222 and the time configuration box 223 enable customization of the patient investigation reports 210.

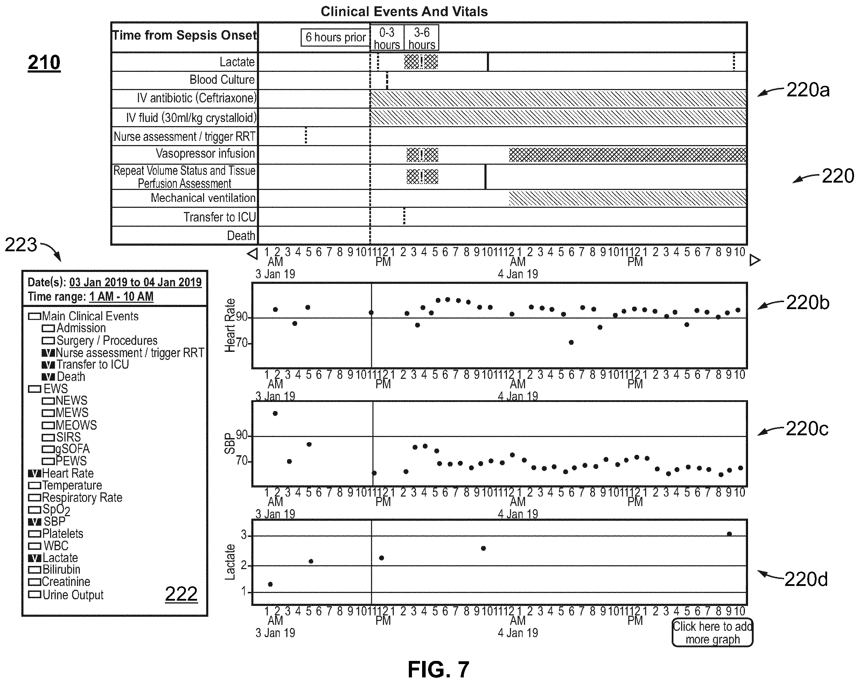

In FIG. 7, the nurse assessment/trigger of RRT, transfer to ICU, and death sub-parameters are selected in the parameter configuration box 222, and are displayed in a chart in a first portion 220a of the display area 220. Also, the heart rate, systolic blood pressure (SBP), and lactate parameters are selected in the parameter configuration box 222, and are respectively displayed in graphs that are included in a second portion 220b, third portion 220c, and fourth portion 220d of the display area 220. Thus, the patient investigation report 210 of FIG. 7 includes multiple charts and graphs that display septic patient data over time.

FIGS. 18, 19, and 26-28 illustrate examples of institutional compliance reports 212. As shown in these figures, the institutional compliance reports 212 include one or more parameter configuration boxes 232 where parameters are selectable for display in one or more graphs and charts in a display area 234. The institutional compliance reports 212 further include a time configuration box 233 where a date and time range are selectable. Accordingly, the institutional compliance reports 212 are customizable based on the selections parameter configuration boxes 232 and time configuration box 233.

The parameter configuration boxes 232 include parameters that define departments or units within an institution such as a hospital. For example, the cardiology department within a hospital may be selected within one of the parameter configuration boxes 232. The parameter configuration boxes 232 also includes parameters that define the severity of sepsis (i.e., sepsis, severe sepsis, and septic shock) and also where the sepsis was acquired (i.e., hospital acquired, community acquired, etc.). Additional parameters may also be selectable within the parameter configuration boxes 232 such as death rate, ICU length of stay, overall length of stay, vasopressor duration, ventilator duration, dialysis duration, extracorporeal membrane oxygenation (ECMO) duration, and readmission rate.

In FIG. 8, a particular department such as the cardiology department and a death rate parameter are selected in the parameter configuration box 232 such that the institutional insights report 214 generates a chart in the display area 234 to display the death rate percentage for patients diagnosed with sepsis, severe sepsis, and septic shock within the selected department of the healthcare facility (e.g., the cardiology department).

In FIG. 9, the institutional insights report 214 displays a graph in the display area 234 that includes the number of patients who acquired sepsis while admitted in a selected department within the healthcare facility and the death rate of the patients over a period of time.

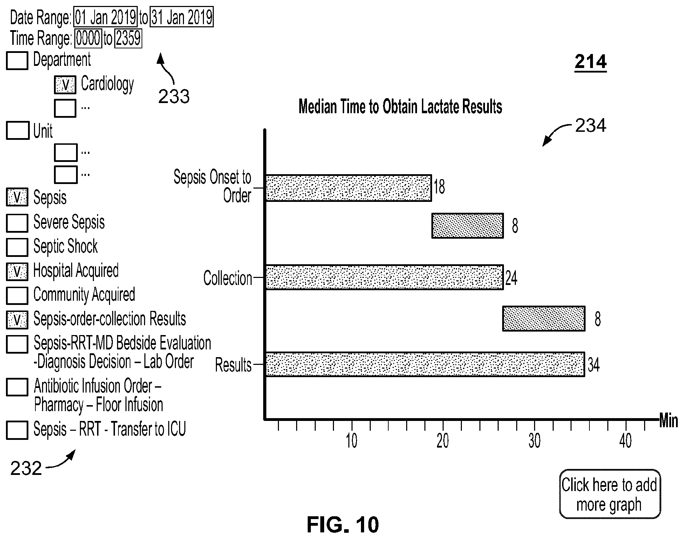

In FIG. 10, the institutional insights report 214 displays a chart in the display area 234 that includes the median time to order, collect, and obtain lactate results.

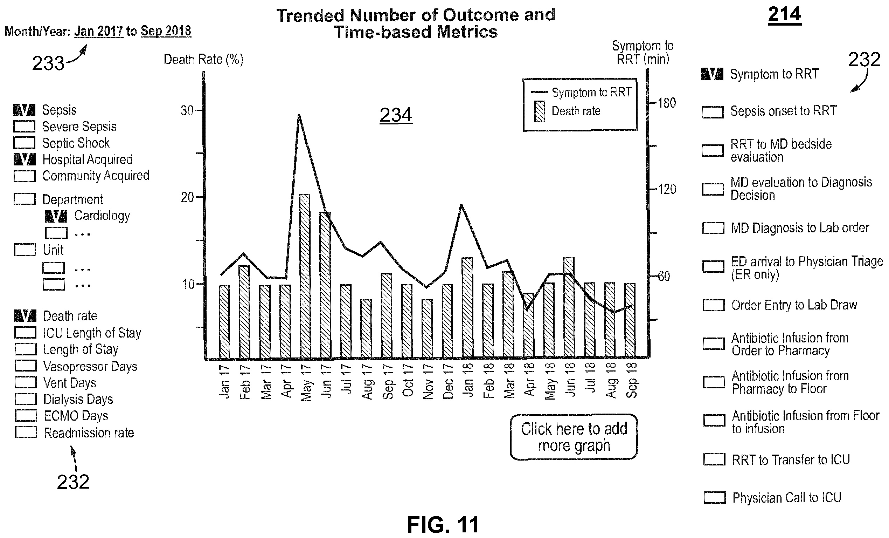

In FIG. 11, the institutional insights report 214 displays a graph in the display area 234 that includes the symptom to rapid response team (RRT) call data and death rate data trended over a period of time for a department within the healthcare facility such as a cardiology department. Additionally, the graph in the institutional insights report 214 of FIG. 11 is for patients diagnosed with sepsis during their stay in the healthcare facility.

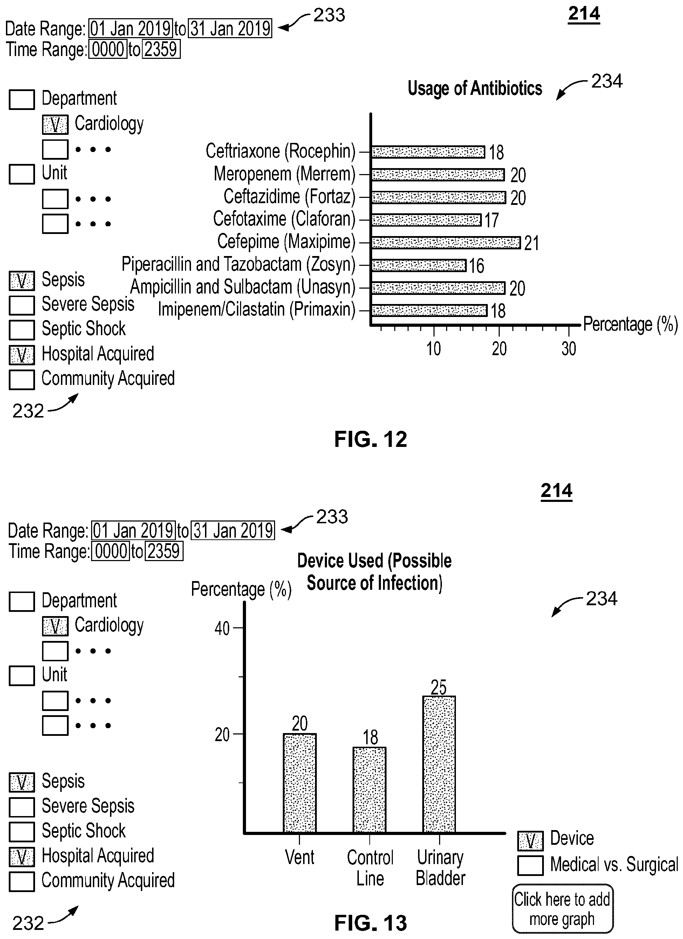

In FIG. 12, the institutional insights report 214 displays a chart in the display area 234 that includes antibiotic usage by septic patients in the cardiology department of the hospital.

In FIG. 13, the institutional insights report 214 displays a chart in the display area 234 that includes the devices used by patients before the onset of sepsis. These devices could be possible sources of the sepsis infection.

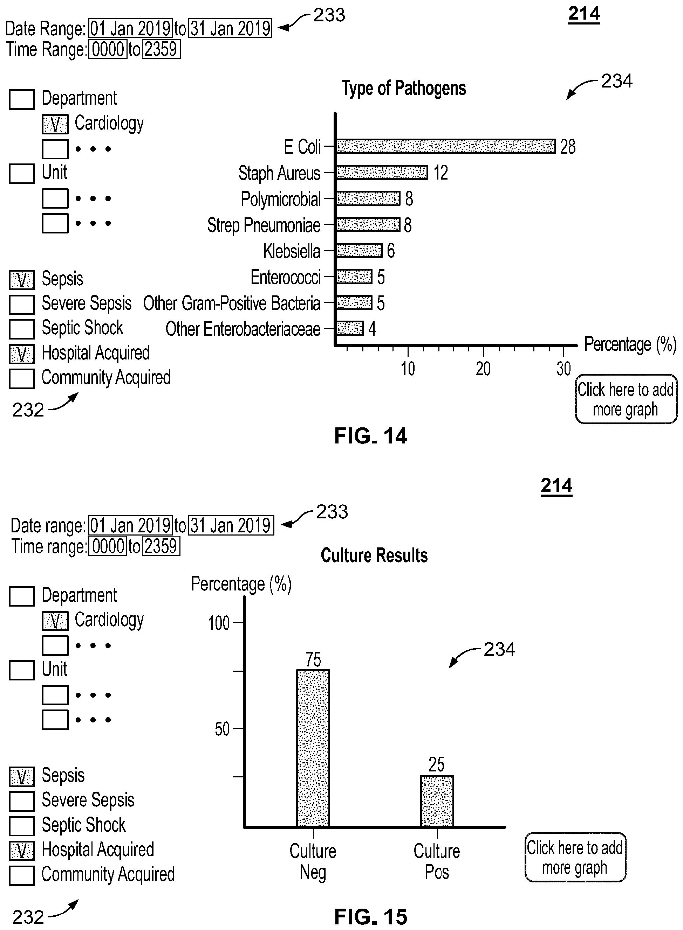

In FIG. 14, the institutional insights report 214 displays a chart in the display area 234 that includes the percentage of patients with specific pathogens determined for hospital acquired sepsis patients in the cardiology department.

In FIG. 15, the institutional insights report 214 displays a chart in the display area 234 that includes the culture results for hospital acquired sepsis patients in the cardiology department.

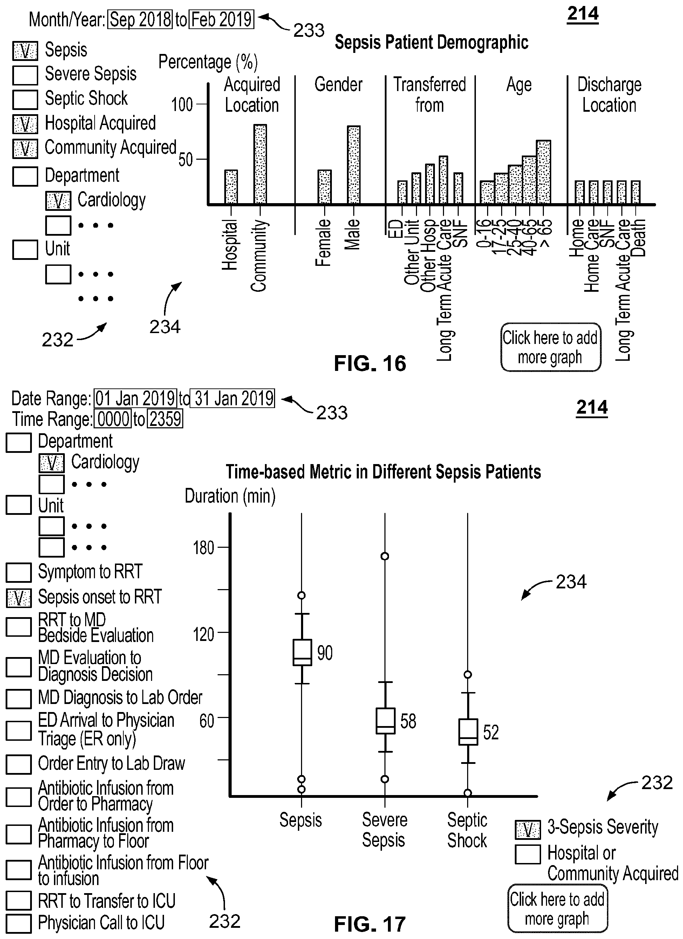

In FIG. 16, the institutional insights report 214 displays in a chart in the display area 234 that includes the sepsis patient demographics for both hospital and community acquired sepsis patients in the cardiology department.

In FIG. 17, the institutional insights report 214 displays a chart in the display area 234 that includes the sepsis onset to rapid response team (RRT) call for patient's diagnosed with sepsis, severe sepsis, and septic shock in the cardiology department of the hospital.

In FIG. 18, the institutional compliance report 212 displays a chart in the display area 234 that includes a comparison of time-based metrics for patients who acquired sepsis while in the cardiology department of the hospital. As shown in FIG. 18, the time-based metrics include emergency department (ED) arrival to physician triage, physician (MD) exam to diagnosis decision, order entry to lab draw, antibiotic infusion from order to pharmacy, antibiotic infusion from pharmacy to floor, antibiotic infusion from floor to infusion, lab order to draw, rapid response team (RRT) call to transfer to ICU, call to ICU, and lab draw to result.

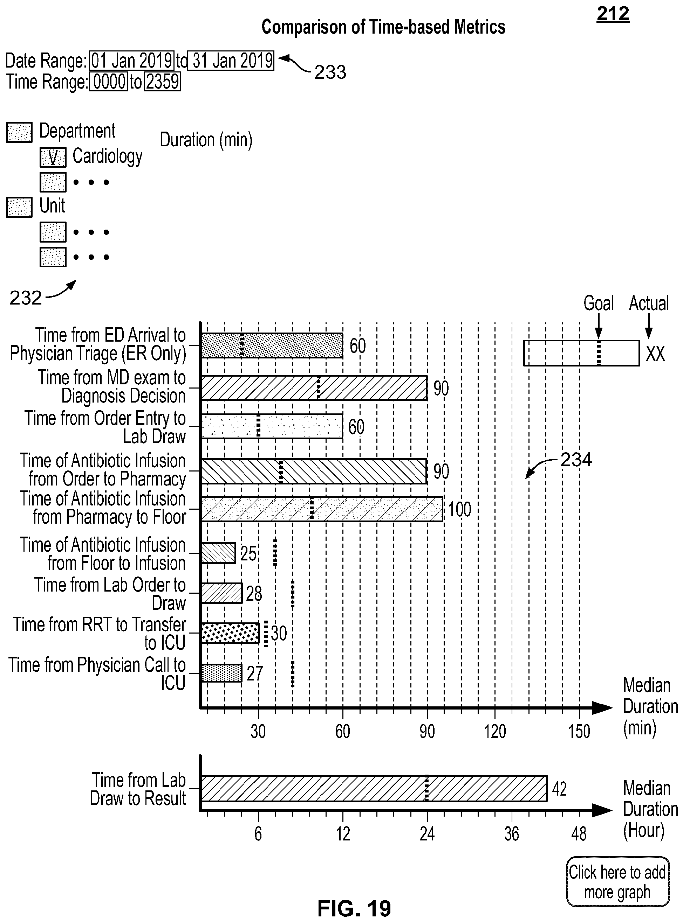

In FIG. 19, the institutional compliance report 212 displays a chart in the display area 234 that includes another comparison of the time-based metrics.

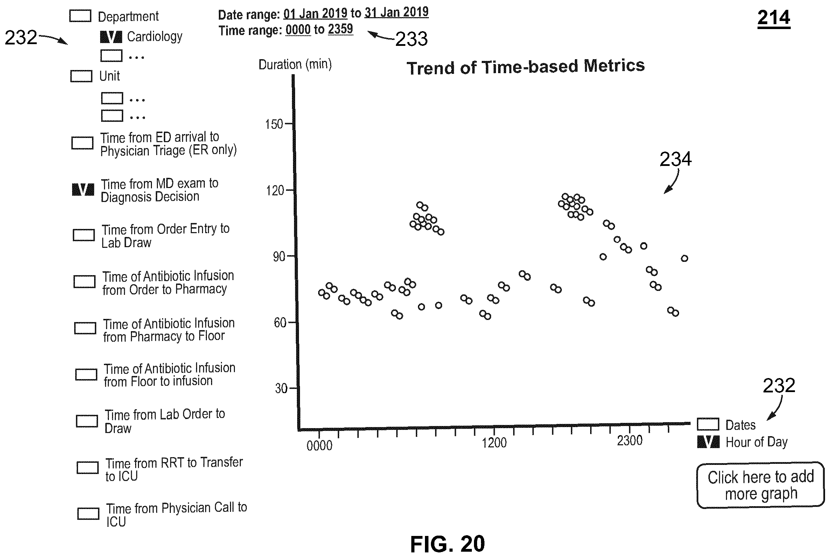

In FIG. 20, the institutional insights report 214 displays a chart in the display area 234 that includes the time from physician (MD) exam to diagnosis decision for septic patients in the cardiology department of the hospital. As shown in FIG. 20, the time-based metrics selected in the parameter configuration boxes 232 are displayed in the display area 234.

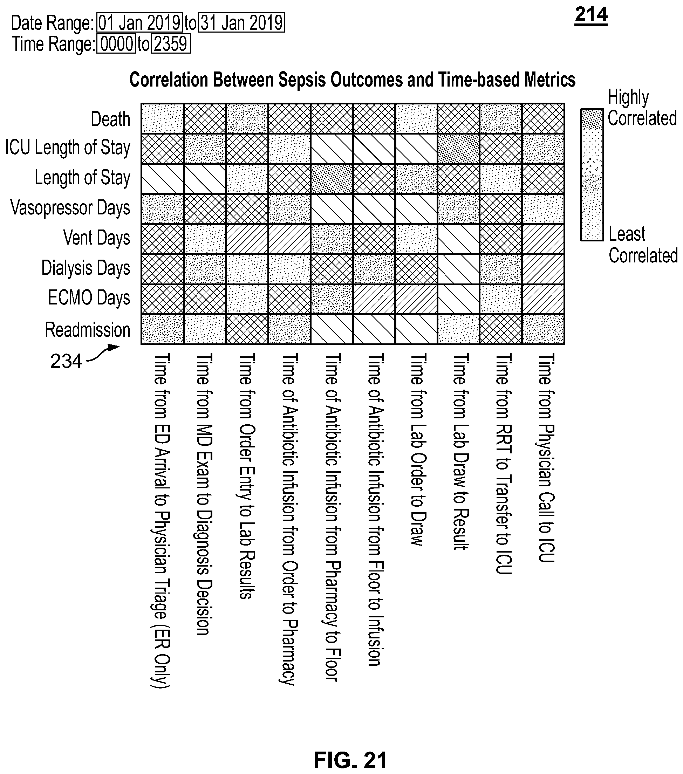

In FIG. 21, the institutional insights report 214 displays a chart in the display area 234 that includes correlations between sepsis outcomes and time-based metrics. In this example, the correlations are color coded such that a first color (e.g., red) depicts a high correlation, a second color (e.g., green) depicts low correlation, and shades of colors between the first and second colors depict degrees of correlation. Sepsis outcomes include death, intensive care unit (ICU) length of stay, total length of stay, vasopressor duration, ventilator duration, dialysis duration, extracorporeal membrane oxygenation (ECMO) duration, and readmission.

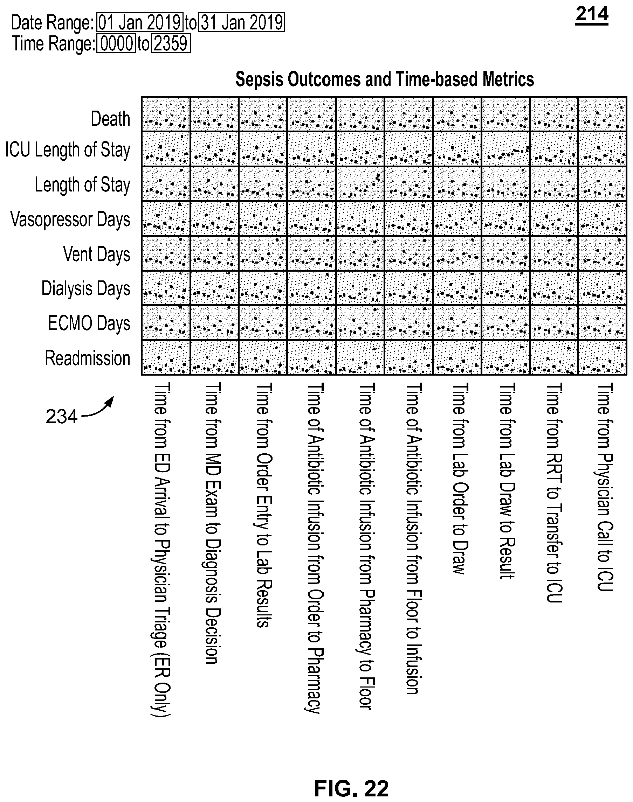

In FIG. 22, the institutional insights report 214 displays a chart in the display area 234 that also includes correlations between sepsis outcomes and time-based metrics. In this example, the correlations are displayed by dots within a box. The dots within each box represent the individual data points upon which the correlation is based (which drives the color of each box). For example, the box in the upper left contains the individual data points for death as a function of time from emergency department arrival to physician triage.

In FIG. 23, the institutional insights report 214 displays a graph in the display area 234 that includes a comparison between length of stay and the time of antibiotic infusion from pharmacy to floor. Different outcomes such as death, ICU length of stay, vasopressor duration, ventilator duration, dialysis duration, ECMO duration, and readmission are selectable in a first parameter configuration box 232 for generating a graph in the display area 234. Additionally, different time-based metrics are selectable in a second parameter configuration box 232 for generating the graph in the display area 234.

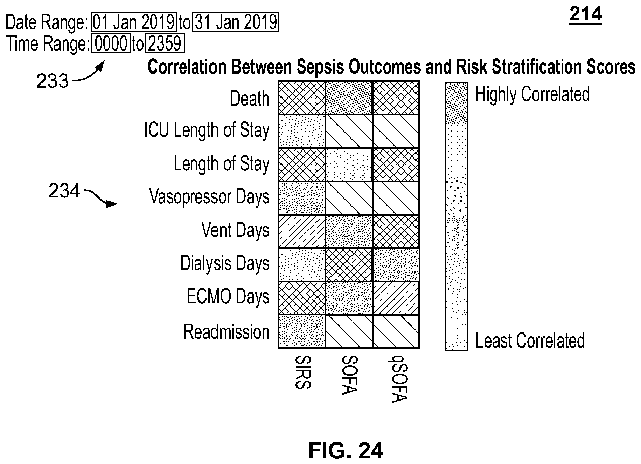

In FIG. 24, the institutional insights report 214 displays a chart in the display area 234 that includes correlations between sepsis outcomes and risk scores.

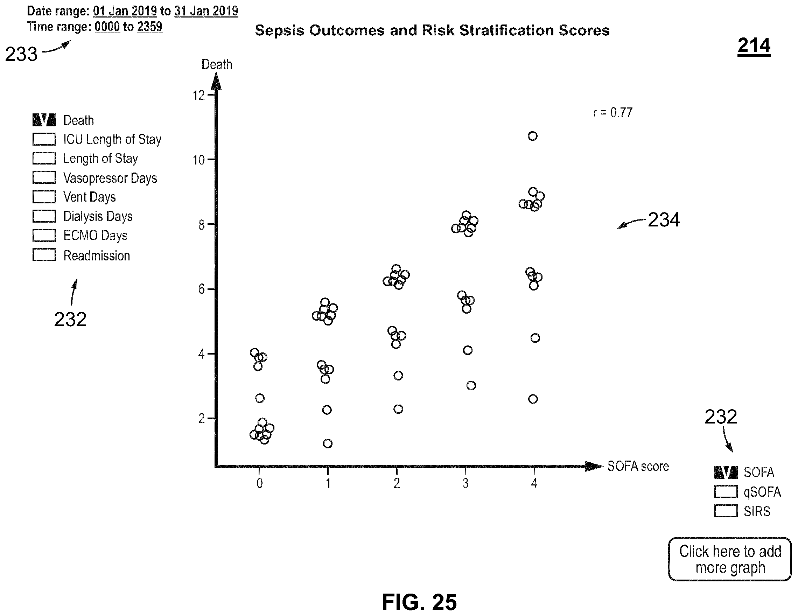

In FIG. 25, the institutional insights report 214 displays a graph in the display area 234 that includes a correlation between death (e.g., a sepsis outcome) and risk scores. Different sepsis outcomes can also be selected in a first parameter configuration box 232 for display in the graph. Additionally, different risk scores can be selected in a second parameter configuration box 232 for display in the graph generated in the display area 234.

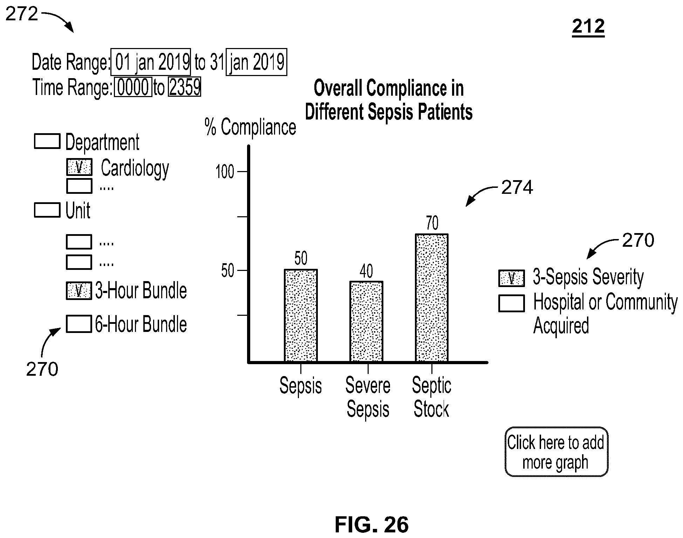

FIGS. 26-28 illustrate examples of institutional compliance reports 212. The institutional compliance reports 212 include one or more parameter configuration boxes 270 where parameters are selectable for one or more graphs and charts in a display area 274. The institutional compliance reports 212 also include a time configuration box 272 where a date and time range are selectable. The parameter configuration boxes 270 and time configuration box 272 enable customization of the institutional compliance reports 212.

The parameter configuration boxes 270 include parameters that define departments or units within an institution such as a hospital. For example, the cardiology department within a hospital may be selected within one of the parameter configuration boxes 270. The parameter configuration boxes 270 also includes sepsis treatment events that are selectable for display such as 3-hr and 6-hr sepsis treatment bundles. The parameter configuration boxes 270 also include additional parameters that define the severity of sepsis (i.e., sepsis, severe sepsis, and septic shock) and also where the sepsis was acquired (i.e., hospital acquired, community acquired, etc.).

In FIG. 26, the institutional compliance report 212 displays a chart in the display area 274 that summarizes compliance for the 3-hr sepsis treatment bundle. The data is categorized by patients diagnosed with sepsis, severe sepsis, and septic shock designated by the selection of the 3-sepsis severity parameter in the parameter configuration boxes 270.

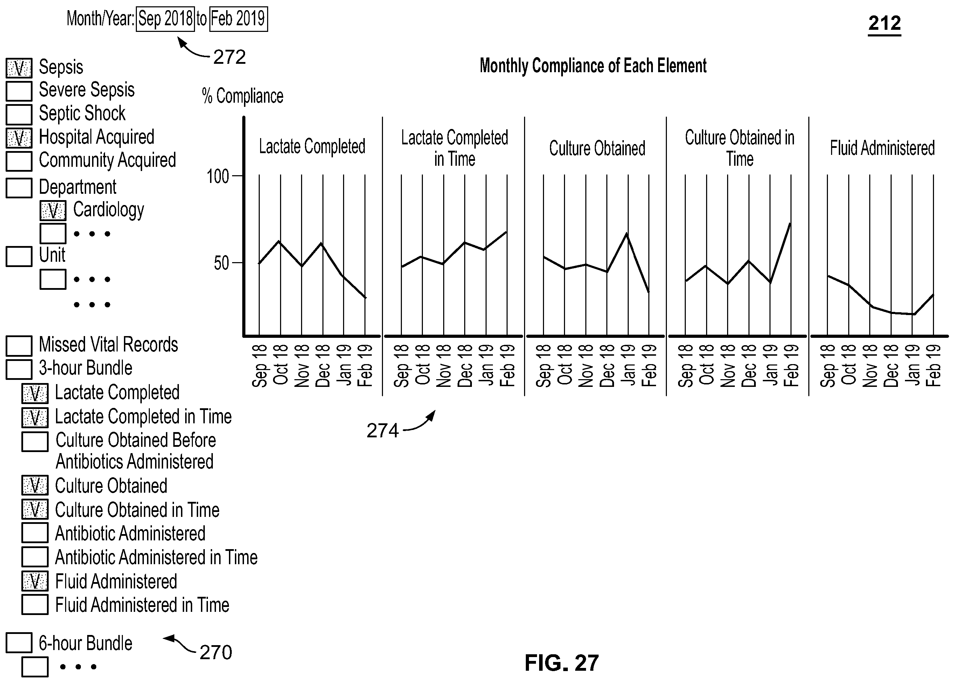

In FIG. 27, the institutional compliance report 212 displays a chart in the display area 274 that has monthly compliance for the 3-hr sepsis treatment bundle per element of the 3-hour sepsis treatment bundle. The chart displays compliance for lactate completed, lactate completed in time, culture obtained, culture obtained in time, and fluid administered. This is designated by the selection of these elements in the parameter configuration boxes 270.

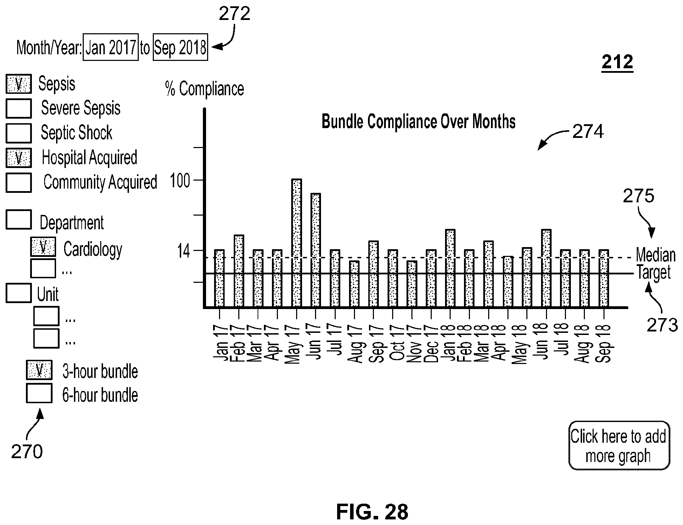

In FIG. 28, the institutional compliance report 212 displays a chart in the display area 274 that includes compliance for the 3-hr sepsis treatment bundle over a period of time (e.g., months). The chart includes a target threshold 273 and a median threshold 275.

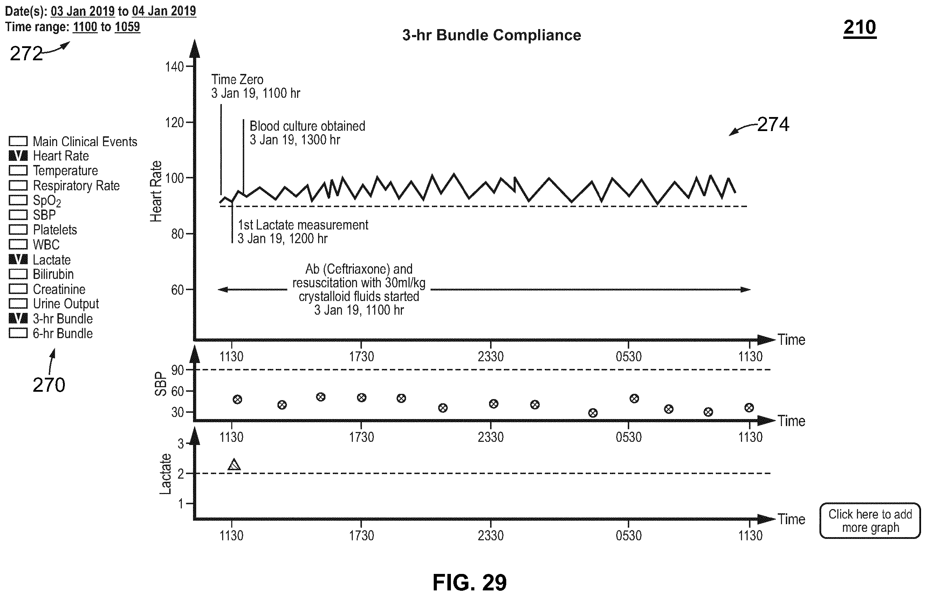

In FIG. 29, the patient investigation report 210 displays a chart in the display area 274 that includes data indicating compliance for the 3-hr sepsis treatment bundle over a period of time for a particular patient. As shown in FIG. 29, the chart also displays data for the patient that is monitored over time such as heart rate, systolic blood pressure (SBP), and lactate.

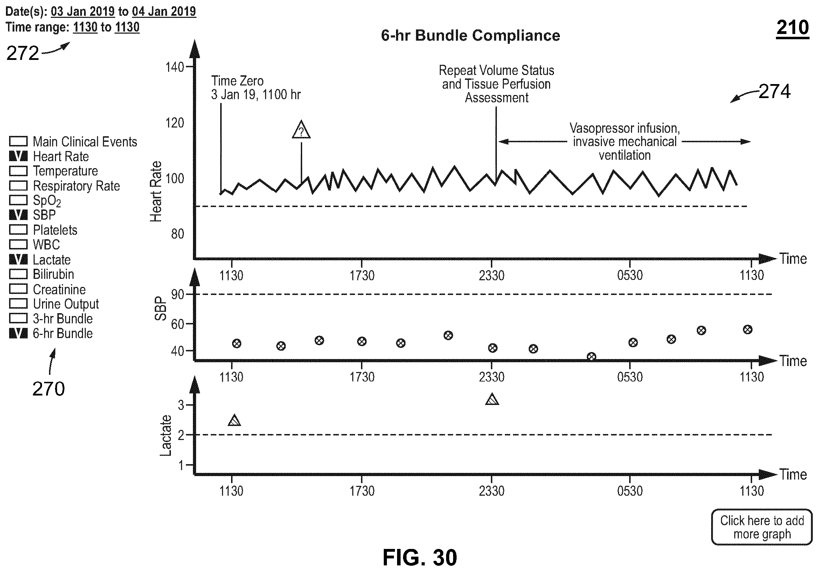

In FIG. 30, the patient investigation report 210 displays a chart in the display area 274 that indicates compliance for the 6-hr sepsis treatment bundle over a period of time for a particular patient. The chart is substantially similar to the chart of FIG. 29, and displays data monitored over time such as heart rate, systolic blood pressure (SBP), and lactate.

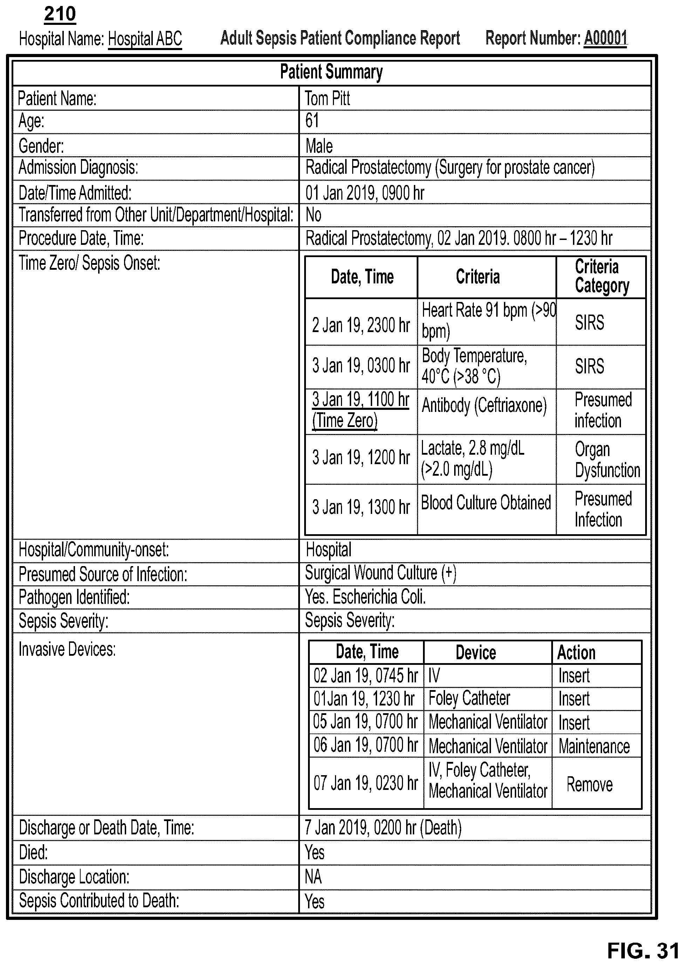

In FIG. 31, the patient investigation report 210 includes patient summary data (e.g., patient name, age, gender, etc.) as well as the time of sepsis onset and criteria used for the diagnosis. The patient investigation report 210 further includes a presumed source of infection, the pathogen identified, sepsis severity, and the invasive devices that were used on the patient. The patient investigation report 210 also includes a date and time of discharge or death, whether the patient died, and whether sepsis contributed to the patient's death.

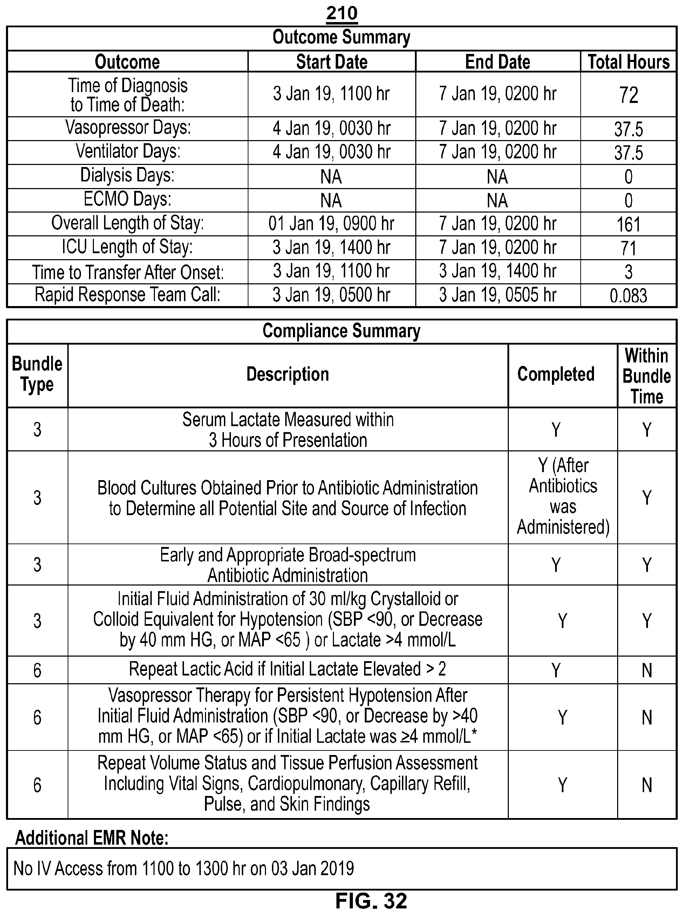

In FIG. 32, the patient investigation report 210 includes an outcome summary that summarizes a start date, end date, and total hours for events such as time of diagnosis to time of death, vasopressor duration, ventilator duration, dialysis duration, ECMO duration, overall length of stay, ICU length of stay, time to transfer after onset, and rapid response team call. The patient investigation report 210 further includes a compliance summary that specifies whether elements of the 3-hr and 6-hr sepsis treatment bundles were completed and within bundle time for a particular patient. The patient investigation report 210 also includes a designated space for caregiver notes (e.g., "no IV access from 1100 to 1300 hr on 03 Jan 2019").

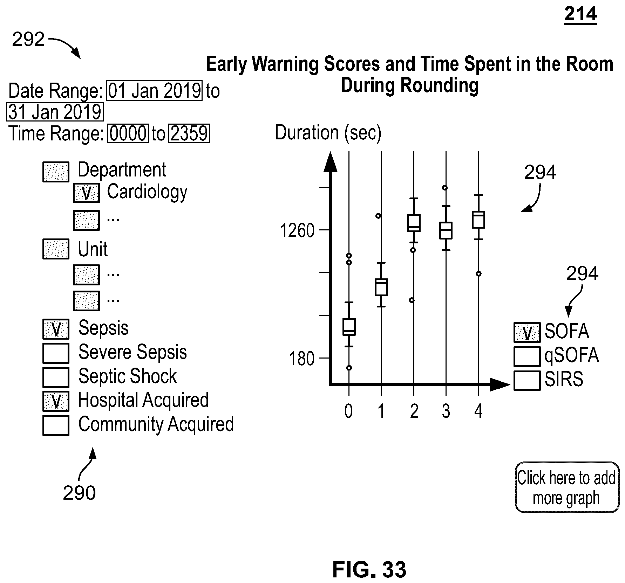

FIGS. 33 illustrates an example institutional insights report 214, while FIGS. 34 and 35 illustrate example sepsis patient investigation reports 210. The institutional insights report 214 include one or more parameter configuration boxes 290 where parameters are selectable for one or more graphs and charts in a display area 294. The institutional insights report 214 also include a time configuration box 292 where a date and time range are selectable. Selection of one or more parameters in the parameter configuration boxes 290 and time configuration box 292 enable customization of the institutional insights report 214.

The parameter configuration boxes 290 include options for selecting a type of early warning score (e.g., NEWS, MEWS, MEOWS, SIRS, SOFA, qSOFA, PEWS, etc.). The parameter configuration boxes 290 also include options for selecting parameters that define departments or units within an institution such as a hospital. Additionally, the parameter configuration boxes 290 also include options for selecting a level of sepsis severity (e.g., sepsis, severe sepsis, and septic shock) and also the location where the sepsis was acquired (e.g., hospital acquired, community acquired, etc.).

In FIG. 33, the institutional insights report 214 displays a chart in the display area 294 that shows time spent in the room during rounding by SOFA early warning scores.

In FIG. 34, the patient investigation report 210 displays a chart in the display area 294 that shows NEWS early warning scores and whether vitals were measured within compliant time interval or were measured beyond the compliant time interval.

In FIG. 35, the patient investigation report 210 displays a chart in the display area 294 that shows time spent in the room during rounding by NEWS early warning scores. As shown in FIGS. 33-35, different early warning scores are selectable for display in the display area 294.

FIG. 36 is a block diagram schematically illustrating physical components (i.e., hardware) of a computing device 1800 with which embodiments of the disclosure may be practiced. In a basic configuration, the computing device 1800 may include at least one processing unit 1802 and a system memory 1804.

Depending on the configuration and type of computing device, the system memory 1804 may comprise, but is not limited to, volatile storage (e.g., random access memory), nonvolatile storage (e.g., read-only memory), flash memory, or any combination of such memories. The system memory 1804 may include an operating system 1805 and one or more program modules 1806 suitable for running software applications 1820. The operating system 1805, for example, may be suitable for controlling the operation of the computing device 1800.

Embodiments of the disclosure may be practiced in conjunction with a graphics library, other operating systems, or any other application program and are not limited to any particular application or system. This basic configuration is illustrated in FIG. 36 by those components within a dashed line 1808. The computing device 1800 may have additional features or functionality. For example, the computing device 1800 may also include additional data storage devices (removable and/or non-removable) such as, for example, magnetic disks, optical disks, or tape. Such additional storage is illustrated by a removable storage device 1809 and a non-removable storage device 1810.

The term computer readable media as used herein may include non-transitory computer storage media. Computer storage media may include volatile and nonvolatile, removable and non-removable media implemented in any method or technology for storage of information, such as computer readable instructions, data structures, or program modules. The system memory 1804, the removable storage device 1809, and the non-removable storage device 1810 are all computer storage media examples (i.e., memory storage). Computer storage media may include RAM, ROM, electrically erasable read-only memory (EEPROM), flash memory or other memory technology, CD-ROM, digital versatile disks (DVD) or other optical storage, magnetic cassettes, magnetic tape, magnetic disk storage or other magnetic storage devices, or any other article of manufacture which can be used to store information and which can be accessed by the computing device 1800. Any such computer storage media may be part of the computing device 1800. Computer storage media does not include a carrier wave or other propagated or modulated data signal.

As stated above, a number of program modules and data files may be stored in the system memory 1804. While executing on the at least one processing unit 1802, the program modules 1806 may perform processes in accordance with embodiments of the present disclosure, and in particular to generate screen content, and may also include electronic mail and contacts applications, word processing applications, spreadsheet applications, database applications, slide presentation applications, drawing or computer-aided application programs, and the like.

The computing device 1800 may also have one or more input device(s) 1812, such as a keyboard, a mouse, a pen, a sound or voice input device, a touch or swipe input device, etc. The output device(s) 1814 such as a display, speakers, a printer, etc. may also be included. The aforementioned devices are examples and others may be used.

The computing device 1800 may include one or more communication connections 1816 allowing communications with other computing devices. Examples of communication connections 1816 include, but are not limited to, RF transmitter, receiver, and/or transceiver circuitry; universal serial bus (USB), parallel, and/or serial ports.

Communication media may be embodied by computer readable instructions, data structures, program modules, or other data in a modulated data signal, such as a carrier wave or other transport mechanism, and include any information delivery media. The term "modulated data signal" may describe a signal that has one or more characteristics set or changed in such a manner as to encode information in the signal. By way of example, communication media may include wired media such as a wired network or direct-wired connection, and wireless media such as acoustic, radio frequency (RF), infrared, and other wireless media.

Embodiments of the present invention may be utilized in various distributed computing environments where tasks are performed by remote processing devices that are linked through a communications network in a distributed computing environment.

The block diagrams depicted herein are just examples. For instance, components may be added, deleted or modified.

The systems and methods described herein provide a technical effect by enabling a computing device to generate customizable sepsis reports from data acquired from systems and devices in a healthcare facility, including electronic medical record systems, hospital information systems, and patient monitoring and support devices, that are not interoperable. Additionally, the acquired data from said systems and devices in the healthcare facility is integrated into a practical application of automatically generating customizable sepsis reports that provide data visualization to track hospital sepsis protocol compliance, investigate septic patient outcomes, prioritize sepsis interventions, and determine areas for improvement in sepsis treatment.

The description and illustration of one or more embodiments provided in this application are not intended to limit or restrict the scope of the invention in any way. The embodiments, examples, and details provided in this application are considered sufficient to convey possession and enable others to make and use the best mode of claimed invention. The invention should not be construed as being limited to any embodiment, example, or detail provided in this application. Regardless whether shown and described in combination or separately, the various features (both structural and methodological) are intended to be selectively included or omitted to produce an embodiment with a particular set of features.

Having been provided with the description and illustration of the present application, one skilled in the art may envision variations, modifications, and alternate embodiments.

## Claims

1. A sepsis automated reporting system (200) comprising:
a communication module (218) configured to acquire data from an electronic medical record system (104), hospital information systems (106), and patient monitoring and support devices (108);
a database storage (202) configured to store the data acquired by the communication module;
at least one processing unit (1802); and
a system memory (1804) storing instructions that, when executed by the at least one processing unit, cause the at least one processing unit to:
generate a sepsis report (210) using the data acquired from the electronic medical record system (104), the hospital information systems (106), and patient monitoring and support devices (108), the sepsis report being accessible on one or more workstations (112), the sepsis report including a display area (220) that is customizable based on a selection of one or more parameters in a parameter configuration box (222), the customizable display area and the parameter configuration box both being displayed in the sepsis report, and the display area being automatically updated based on the selection of the one or more parameters, wherein the sepsis report is a patient investigation report (210) that includes data from a single, septic patient in a healthcare facility (100) and wherein the patient investigation report enables selection of a vital sign parameter and clinical events in the parameter configuration box to generate a graph in the display area displaying the vital sign parameter and the clinical events over a period of time, wherein the vital sign parameter is a heart rate, a temperature, a respiratory rate, an SpO₂, or a systolic blood pressure.

2. The system of claim 1, wherein the sepsis automated reporting system is cloud based and is accessible by a web portal that provides a single sign-on configuration application.

3. The system of claim 1, wherein the sepsis automated reporting system is accessible by an intranet portal of a local area network that provides a single sign-on configuration application.

4. The system of any preceding claim, wherein the hospital information systems include an admission, discharge, and transfer system (111), a lab system (113), a medication system (115), a nurse call system (105), a real-time locating system (107), and a mobile device platform (109).

5. The system of any preceding claim, wherein the patient monitoring and support devices (108) include at least one of a vital signs monitor, a bed, and a mattress pad device.

6. The system of any preceding claim, wherein the sepsis report is accessible via a plurality of workstations within a healthcare facility.

7. The system of any preceding claim, wherein the communication module (218) acquires the data using a Health Level Seven International messaging protocol.

8. The system of any preceding claim, wherein the sepsis report further includes a time configuration box (223) where a time range is selectable.

9. The system of claim 8, wherein selections made in the parameter configuration box and the time configuration box enable customization of the sepsis reports.

## Patentansprüche

1. Automatisiertes Sepsismeldesystem (200), umfassend:
ein Kommunikationsmodul (218), ausgebildet zum Erfassen von Daten aus einem elektronischen Patientenaktensystem (104), Krankenhausinformationssystemen (106) und Patientenüberwachungs- und -unterstützungsvorrichtungen (108);
einen Datenbankspeicher (202), ausgebildet zum Speichern der durch das Kommunikationsmodul erfassten Daten;
mindestens eine Verarbeitungseinheit (1802); und
einen Systemspeicher (1804), in dem Anweisungen gespeichert sind, die, wenn sie durch die mindestens eine Verarbeitungseinheit ausgeführt werden, die mindestens eine Verarbeitungseinheit veranlassen zum:
Erstellen eines Sepsisberichts (210) unter Verwendung der Daten, die aus dem elektronischen Patientenaktensystem (104), den Krankenhausinformationssystemen (106) und den Patientenüberwachungs- und -unterstützungsvorrichtungen (108) erfasst wurden, wobei der Sepsisbericht an einer oder mehreren Arbeitsstationen (112) zugänglich ist, wobei der Sepsisbericht einen Anzeigebereich (220) einschließt, der auf der Grundlage einer Auswahl von einem oder mehreren Parametern in einem Parameterkonfigurationsfeld (222) individuell anpassbar ist, wobei sowohl der individuell anpassbare Anzeigebereich als auch das Parameterkonfigurationsfeld in dem Sepsisbericht angezeigt sind und der Anzeigebereich auf der Grundlage der Auswahl des einen oder der mehreren Parameter automatisch aktualisiert wird, wobei der Sepsisbericht ein Patientenuntersuchungsbericht (210) ist, der Daten von einem einzelnen Sepsispatienten in einer Gesundheitspflegeeinrichtung (100) einschließt, und wobei der Patientenuntersuchungsbericht die Auswahl eines Vitalzeichenparameters und klinischer Ereignisse in dem Parameterkonfigurationsfeld ermöglicht, um ein Diagramm in dem Anzeigebereich zu erstellen, das den Vitalzeichenparameter und die klinischen Ereignisse über einen Zeitraum anzeigt, wobei es sich bei dem Vitalzeichenparameter um eine Herzfrequenz, eine Temperatur, eine Atemfrequenz, einen SpO₂ oder einen systolischen Blutdruck handelt.

2. System nach Anspruch 1, wobei das automatisierte Sepsismeldesystem cloudbasiert ist und über ein Webportal zugänglich ist, das eine Single-Sign-On-Konfigurationsanwendung bereitstellt.

3. System nach Anspruch 1, wobei das automatisierte Sepsismeldesystem über ein Intranet-Portal eines lokalen Netzwerks zugänglich ist, das eine Single-Sign-On-Konfigurationsanwendung bereitstellt.

4. System nach einem vorstehenden Anspruch, wobei die Krankenhausinformationssysteme ein Aufnahme-, Entlassungs- und Verlegungssystem (111), ein Laborsystem (113), ein Medikationssystem (115), ein Schwesternrufsystem (105), ein Echtzeit-Ortungssystem (107) und eine Plattform (109) für mobile Vorrichtungen einschließen.

5. System nach einem vorstehenden Anspruch, wobei die Patientenüberwachungs- und -unterstützungsvorrichtungen (108) mindestens eines einschließen von einer Vitalzeichenüberwachungsvorrichtung, einem Bett und einer Matratzenauflagevorrichtung.

6. System nach einem vorstehenden Anspruch, wobei der Sepsisbericht über eine Mehrzahl von Arbeitsstationen innerhalb einer Gesundheitspflegeeinrichtung zugänglich ist.

7. System nach einem vorstehenden Anspruch, wobei das Kommunikationsmodul (218) die Daten unter Verwendung eines Health-Level-Seven-International-Meldungsprotokolls erfasst.

8. System nach einem vorstehenden Anspruch, wobei der Sepsisbericht weiter ein Zeitkonfigurationsfeld (223) einschließt, in dem ein Zeitbereich auswählbar ist.

9. System nach Anspruch 8, wobei die in dem Parameterkonfigurationsfeld und dem Zeitkonfigurationsfeld getroffenen Auswahlen die individuelle Anpassung der Sepsisberichte ermöglichen.

## Revendications

1. Système (200) de signalement automatisé de septicémie comprenant :
un module de communication (218) configuré pour acquérir des données à partir d'un système (104) de dossiers médicaux électroniques, de systèmes d'information hospitaliers (106) et de dispositifs (108) de surveillance et de support de patients ;
un stockage (202) de base de données configuré pour stocker les données acquises par le module de communication ;
au moins une unité de traitement (1802) ; et
une mémoire système (1804) stockant des instructions qui, lorsqu'elles sont exécutées par l'au moins une unité de traitement, amènent l'au moins une unité de traitement à :
générer un rapport (210) de septicémie en utilisant les données acquises à partir du système (104) de dossiers médicaux électroniques, des systèmes d'information hospitaliers (106) et des dispositifs (108) de surveillance et de support de patients, le rapport de septicémie étant accessible sur un ou plusieurs postes de travail (112), le rapport de septicémie incluant une zone d'affichage (220) qui est personnalisable en fonction d'une sélection d'un ou plusieurs paramètres dans une boîte (222) de configuration de paramètres, la zone d'affichage personnalisable et la boîte de configuration de paramètres étant toutes les deux affichées dans le rapport de septicémie, et la zone d'affichage étant automatiquement mise à jour en fonction de la sélection du ou des paramètres, dans lequel le rapport de septicémie est un rapport d'investigation (210) de patient qui inclut des données d'un seul patient présentant une septicémie dans un établissement de santé (100) et dans lequel le rapport d'investigation de patient permet la sélection d'un paramètre de signe vital et d'événements cliniques dans la boîte de configuration de paramètres pour générer un graphique dans la zone d'affichage affichant le paramètre de signe vital et les événements cliniques sur une période de temps, dans lequel le paramètre de signe vital est une fréquence cardiaque, une température, une fréquence respiratoire, un SpO₂, ou une pression systolique.

2. Système selon la revendication 1, dans lequel le système de signalement automatisé de septicémie est basé sur le cloud et est accessible via un portail web qui fournit une application de configuration à authentification unique.

3. Système selon la revendication 1, dans lequel le système de signalement automatisé de septicémie est accessible par un portail intranet d'un réseau local qui fournit une application de configuration à authentification unique.

4. Système selon une quelconque revendication précédente, dans lequel les systèmes d'information hospitaliers incluent un système (111) d'admission, de sortie et de transfert, un système (113) de laboratoire, un système de médication (115), un système (105) d'appel d'infirmier, un système de localisation en temps réel (107) et une plateforme de dispositif mobile (109).

5. Système selon une quelconque revendication précédente, dans lequel les dispositifs (108) de surveillance et de support de patient incluent au moins un élément parmi un moniteur de signes vitaux, un lit et un dispositif d'alèse de matelas.

6. Système selon une quelconque revendication précédente, dans lequel le rapport de septicémie est accessible via une pluralité de postes de travail au sein d'un établissement de santé.

7. Système selon une quelconque revendication précédente, dans lequel le module de communication (218) acquiert les données en utilisant un protocole de messagerie Health Level Seven International.

8. Système selon une quelconque revendication précédente, dans lequel le rapport de septicémie inclut en outre une boîte de configuration temporelle (223) où une plage temporelle peut être sélectionnée.

9. Système selon la revendication 8, dans lequel les sélections effectuées dans la boîte de configuration de paramètres et la boîte de configuration temporelle permettent la personnalisation des rapports de septicémie.
